(19) Europäisches Patentamt
European Patent Office
Office européen des brevets

(11) EP 0 563 557 B1

(12) **EUROPÄISCHE PATENTSCHRIFT**

(45) Veröffentlichungstag und Bekanntmachung des Hinweises auf die Patenterteilung:
**12.08.1998 Patentblatt 1998/33**

(51) Int Cl.6: **C07C 43/13**, C07C 215/08, H01L 39/24

(21) Anmeldenummer: **93102398.0**

(22) Anmeldetag: **16.02.1993**

(54) **Flüchtige Seltenerdmetall Alkoxide, insbesondere zur Herstellung von Seltenerdmetall Oxiden, sowie Alkohole zum Synthetisieren von flüchtigen Verbindungen**

Volatile rate earth metal alkoxides, especially for the preparation of rare earth metal oxides, and alcohols for the synthesis of volatile compounds

Alkoxides volatiles de terres rares, particulierement pour la préparation d'oxydes de terres rares, et des alcohols pour la synthèse de composés volatiles

(84) Benannte Vertragsstaaten:
**DE FR GB IT NL**

(30) Priorität: **18.03.1992 DE 4208689**

(43) Veröffentlichungstag der Anmeldung:
**06.10.1993 Patentblatt 1993/40**

(73) Patentinhaber: **Patent-Treuhand-Gesellschaft für elektrische Glühlampen mbH**
**81543 München (DE)**

(72) Erfinder:
- **Wolfgang, Hermann, Prof.Dr.**
  **W-8050 Freising (DE)**
- **Reiner, Anwander**
  **W-8000 München 2 (DE)**

(56) Entgegenhaltungen:
**EP-A- 0 244 917** **EP-A- 0 256 896**
**WO-A-89/07666** **CA-A- 2 002 606**

- **AMERICAN INSTITUTE OF PHYSICS CONFERENCE PROCEEDINGS Nr. 200 , 1990 , NEW YORK Seiten 157 - 164 J. H. WANDASS ET AL 'APPLICATION OF SOL-GEL TECHNIQUES TO THIN-FILM SUPERCONDUCTOR SYSTEMS' 'CHEMICAL ABSTRACTS TWELFTH COLLECTIVE INDEX, CHEMICAL SUBSTANCES' , AMERICAN CHEMICAL SOCIETY**
- **BULLETIN OF THE CHEMICAL SOCIETY OF JAPAN Bd. 52, Nr. 6 , Juni 1979 , TOKYO JP Seiten 1873 - 1874 M. INOUE ET AL 'FRIEDEL-CRAFTS REACTION OF ANISOLE WITH 2-t-BUTYLOXIRANE'**
- **BERICHTE DER DEUTSCHEN CHEMISCHEN GESELLSCHAFT Bd. 125, Nr. 11 , November 1992 , WEINHEIM DE Seiten 2399 - 2405 W. A. HERRMANN ET AL 'FLÜCHTIGE NEODYM- UND YTTRIUM-ALKOXIDE MIT NEUEN SPERRIGEN CHELATLIGANDEN'**
- **Lehrbuch der organischen Chemie, Beyer, 1967, S. 213**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 563 557 B1

Printed by Jouve, 75001 PARIS (FR)

## Beschreibung

Die Erfindung geht aus von flüchtigen Seltenerdmetall-Alkoxiden gemäß dem Oberbegriff des Anspruchs 1.

Flüchtige Alkoxide spielen als molekulare Vorstufen für neue anorganische Materialien, die aus Ein- oder Mehrkomponentenoxiden bestehen, eine große Rolle (New Journal of Chemistry 11, 1987, S. 663-675). Beispielsweise lassen sich damit Keramiken, dünne Schichten, Katalysatoren oder Membrane herstellen. Für viele Anwendungen ist es dabei wichtig, daß die Oxide möglichst frei von Verunreinigungen sind. Diese Eigenschaft ist beispielsweise bei elektronischen Materialien, insbesondere supraleitenden Keramiken, besonders wichtig. Man ist daher in diesem Fall auf nichtwässrige Lösungen angewiesen, um ionische Verunreinigungen zu vermeiden.

Als besonders geeignete Vorstufen für die Darstellung von Seltenerdmetall-Oxiden haben sich bisher β-Diketonat-Komplexe dieser Metalle erwiesen.

Unter den Seltenerdmetallen sind im folgenden die Elemente Sc, Y, La und die Lanthanoide Ce bis Lu zu verstehen. Bei Verwendung von Alkoxiden als Vorstufe tritt bisher die Schwierigkeit auf, daß die Derivate polymerisieren und kinetische Instabilitätsprobleme entstehen.

Die Abscheidung der Oxide aus den Vorstufen zur Herstellung dünner Schichten erfolgt üblicherweise entweder naßchemisch im Sol-Gel-Verfahren oder aus der Gasphase mittels MOCVD-Technologie (Metal Organic Chemical Vapour Deposition). Insbesondere für diese Abscheidung aus der Gasphase spielt die Flüchtigkeit der jeweiligen Vorstufen-Verbindung neben ihrer Löslichkeit eine entscheidende Rolle. Seltenerdmetall-Alkoxide sind entweder heteroleptische Komplexe oder insbesondere homoleptische Verbindungen, die üblicherweise der Formel $M(OR)_3$ entsprechen. M steht für das Seltenerdmetall, R für einen organischen Rest. Bisher finden hauptsächlich Alkoxide unter ausschließlicher Verwendung von Kohlenwasserstoffen ("CH") als organischen Resten Beachtung, die man auch als M-(CH)-Alkoxide bezeichnet.

Bei der stabilsten Oxidationsstufe III von M werden drei Liganden benötigt. Dadurch treten erhebliche Probleme mit der Koordinationssphäre auf, die die Anwendung von Alkoxiden auf den ersten Blick als aussichtslos erscheinen lassen. Es hat sich nämlich gezeigt, daß die Benutzung von (CH)-Alkoxiden, die von einfachen Alkoholen abgeleitet sind, nicht zum Erfolg führt, weil sie entweder Oligomere (bei Ableitung aus $HOCH_3$ und $HOC_2H_5$) oder Cluster (bei Ableitung aus $HO^iPr$ und $HO^tBu$) bilden und daher in aliphatischen Kohlenwasserstoffen unlöslich und schwerflüchtig sind.

Sterisch anspruchsvollere Alkohole, wie "H-Tritox" und "H-Silox" (Tris(tert.-butyl)methanol bzw. -silanol) ergeben zwar endlich die gewünschten Monomere, die dementsprechend in aliphatischen Kohlenwasserstoffen gut löslich sind. Sie eignen sich daher ausgezeichnet für den Sol-Gel-Prozeß (Chem. Rev. 89, 1989, S. 1317-1322), aber ihre Anwendung für die MOCVD-Technik scheitert, da sie nicht oder nur sehr schwer in die Gasphase gebracht werden können. Ursache dieser Schwierigkeit ist der sog. "CH-bulk". Dieser Ausdruck bezeichnet die Möglichkeit, daß ein H-Atom und insbesondere das β-Alkyl innerhalb des Liganden wandert. Es hat sich gezeigt, daß die thermische Stabilität des (CH)-Alkoxids sehr stark von der Zahl der C- und H-Atome im Alkoxy-Liganden beeinflußt wird. Diese Anhäufung von C- und H-Atomen muß deshalb so weit abgebaut werden, daß das M-Alkoxid gerade noch monomer ist. Es muß daher im Prinzip ein Austausch von Methylgruppen durch H-Atome im Liganden erfolgen. Entsprechende Versuche sind bisher im Fall der Seltenerdmetalle jedoch erfolglos geblieben.

Um den störenden "CH-bulk" abzubauen, wurden als Alternative zu den elementaren (CH)-Alkoxiden daher auch sog. (CHX)-Alkoxide untersucht. Der Aufbau dieser sterisch anspruchsvollen Alkohole erfolgt durch systematischen Austausch der Methyl-Gruppen im Tritox-Derivat durch andere Atome X. Bei Verwendung von Fluoratomen (X=F) erhält man die bisher flüchtigsten M-Alkoxide (Coord. Chem. Rev. 81, 1988, S. 133-202).

Eine zweite Alternative erhält man, wenn man in Anlehnung an den sechsteiligen Ring der β-Diketonate Donorfunktionalitäten (Do) in den CH-Liganden einführt, so daß sog. (CHDo)-Alkoxide gebildet werden. Im einfachsten Fall ergibt sich damit ein quasi durch die freien Elektronenpaare des Donoratoms Do abgeschlossener fünfteiliger Ring mit folgender Struktur eines am Seltenerdmetall M angekoppelten Liganden mit "organischen" (einschließlich H-Atomen) Resten $R_1$-$R_4$ (zusammenfassend als $R_c$ bezeichnet, da an Kohlenstoffatome gebunden) bzw. organischen Resten $R_{Do}$ (an das Donoratom gebunden):

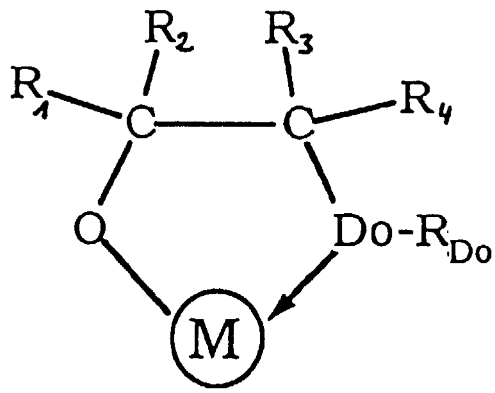

(1)

Im folgenden soll für den Liganden die Kurzschreibweise $O-CR_1R_2-CR_3R_4-Do-R_{Do}$ verwendet werden. Z.B. wird für homoleptische Alkoxide des Typs M $(OR)_3$ dieser Ligand dementsprechend dreimal eingesetzt.

Bisherige Versuche, bei Seltenerdmetallen derartige (CHDo)-Liganden einzusetzen, sind bislang nur im Hinblick auf den Sol-Gel-Prozeß durchgeführt worden (Inorg. Chem. 29, 1990, S. 2883-2885) und führten nur zu dem nicht flüchtigen oligomeren Seltenerd-Komplex $(Y(OCH_2CH_2OCH_3)_3)_{10}$.

Andererseits ist es bisher lediglich gelungen, als Metallzentren die Hauptgruppenmetalle Bi (III) und Pb (II) und die äußeren Übergangsmetalle (d-Metalle) Zn (II) und Cu (II) in flüchtige (CHDo)-Alkoxide einzubauen, nicht jedoch Metalle der Seltenen Erden (J. Am. Chem. Soc. 113, 1991, 1844-1845 und Polyhedron 9, 1990, 611-613 sowie Polyhedron 10, 1991, 437-445).

Im Unterschied zu den bereits verwendeten äußeren Übergangsmetallen, die zum einen zweiwertig sind (Koordinationszahl vier) und einen sehr kleinen effektiven Kationenradius besitzen (typisch 0,6 A), handelt es sich bei den Seltenerdmetallen um innere Übergangsmetalle (f-Metalle einschließlich Y, La), die für die Komplexbildung wesentlich ungünstigere Voraussetzungen aufweisen.

Ein wesentlicher Unterschied zu dem dreiwertigen Hauptgruppenelement Wismut besteht in der Oxophilie der Elemente (sie ist mit bedingt durch die unterschiedlichen Elektronegativitäten: Bi 1.67; Seltenerdmetalle 1.06 - 1.20). Dies zeigt sich schon im Vorkommen der Elemente in der Natur: Während die Lanthanoide ausschließlich in sauerstoffhaltigen Erzen zu finden sind, trifft man Wismut auch als Wismutglanz $Bi_2S_3$ (mit weichem Gegenion $S^{2-}$) an. Des weiteren wird die unterschiedliche Affinität zu Sauerstoff verstärkt deutlich, wenn man die Bildungsenthalpien für das Metalloxid $M_2O_3$ betrachtet (Bi -574,3 kJ/mol; Seltenerdmetalle typisch -1800 bis -1900 kJ/mol).

Die Donorbindung zu den "harten" Seltenerdmetallen sollte daher erheblich stärker sein. Die Stärke der Donorbindung hat entscheidenden Einfluß auf die thermische Stabilität des Alkoxidkomplexes, d.h. ob der Komplex unzersetzt sublimierbar ist.

Auf die thermische Labilität von Wismutkomplexen mit CHDo-Alkoholen wird in Inorg. Chem. 29, 1990, S. 358-360, ausdrücklich hingewiesen. Auch sind β-Diketonat-Komplexe von Wismut erst seit kurzem bekannt (Polyhedron 10, 1991, S. 437-445). Als flüchtige Vorstufe für "1-2-3-Supraleiter" wurde als Bi-Komponente bisher ausschließlich Wismuttriphenyl verwendet.

Aus der CA-A-2 002 606 sind lösliche Seltenerdmetall-Alkoxide bekannt, die für den Sol-Gel-Prozeß geeignet sind. Kennzeichnend ist eine relativ niedrige sterische Beanspruchung, wobei organische Reste, die keine H-Atome sind, nur am Cβ- oder Cγ-Atom zu finden sind.

Die Schrift American Institute of Physics Conference Proceedings, Nr. 200, 1990, S. 157-164, beschreibt ebenfalls Seltenerdmetall-Alkoxide hoher Löslichkeit, die für den Sol-Gel-Prozeß geeignet sind. Als Beispiel ist Yttriummethoxipropoxid (Atomgewicht 74) genannt. Von einer Anwendung für CVD wird abgeraten.

In den Berichten der Deutschen Chemischen Gesellschaft, 125, November 1992, S. 2399 - 2405, ist der Inhalt des Prioritätsdokuments unter der Bezeichnung "Flüchtige Neodym- und Yttrium-Alkoxide mit neuen sperrigen Chelatliganden" wiedergegeben.

Es ist Aufgabe der vorliegenden Erfindung, flüchtige Seltenerdmetall-Alkoxide, insbesondere zur Herstellung von Seltenerdmetall-Oxiden, bereitzustellen, die als Monomere vorliegen und bei niedrigen Temperaturen, insbesondere höchstens 175 °C, bevorzugt weniger als 155 °C, unzersetzt in die Gasphase gebracht werden können und die sich außerdem erst bei Temperaturen über 200 °C zersetzen. Die Temperaturen beziehen sich dabei auf die Sublimation des festen Stoffes bei einem Druck von $10^{-3}$ mbar.

Diese Aufgabe wird durch die kennzeichnenden Merkmale des Anspruchs 1 gelöst. Bevorzugte Ausführungen der Alkoxide sowie ein vorteilhafter Weg zur Herstellung der Oxide finden sich in den darauf gerichteten Unteransprüchen.

Eine weitere Aufgabe der vorliegenden Erfindung ist es, Alkohole bereitzustellen, die sich besonders gut für die

Darstellung der Liganden von flüchtigen und löslichen Metall-Alkoxiden eignen. Insbesondere sollen sie für Seltenerdmetall-Alkoxide geeignet sein.

Diese Aufgabe wird durch die kennzeichnenden Merkmale des Anspruchs 16 gelöst.

Die erfindungsgemäßen Alkoxide eignen sich hervorragend sowohl für den Sol-Gel-Prozeß als auch für die MOCVD-Technik. Der besondere Vorteil der MOCVD-Technik mit Alkoxiden liegt darin, daß ein zusätzliches Reagens für die Abscheidung zum Oxid nicht unbedingt benötigt wird. Hingegen ist bei β-Diketonaten ein MOCVD-Prozeß nur unter Verwendung einer zusätzlichen reaktiven Gaskomponente (z.B. NO, "THF") möglich.

Die Bedeutung der vorliegenden Erfindung liegt in folgendem Umstand:
Im Gegensatz zu den eingangs diskutierten Komplexen der äußeren Übergangsmetalle, sind die Atome der Seltenerdmetalle sehr groß, typische Werte des effektiven Ionenradius liegen bei 0,85-1,05 A, und weisen für komplexe Verbindungen eine hohe Koordinationszahl (bevorzugt sechs oder größer) auf. Das Problem bei der Synthese ihrer Alkoxide ist deren ausgeprägte Oligomerisierungstendenz. Verbindungen sind aber nur dann flüchtig, wenn sie einen niedrigen Oligomerisierungsgrad besitzen (bevorzugt monomer). Die Forschung ist bisher andere Wege gegangen, um flüchtige Vorstufen für Seltenerdmetalloxide herzustellen (β-Diketonate), da die Erfolgsaussichten bei der Verwendung von Alkoxiden sehr gering erschienen.

Überraschenderweise ist es mit der vorliegenden Erfindung jedoch gelungen, eine Gruppe von Alkoxy-Liganden zu finden, die einen optimalen Kompromiß zwischen den verschiedenen Randbedingungen ermöglichen.

Das Zusammenwirken der organischen Reste $R_c$ und $R_{Do}$ ist dahingehend optimiert, daß sie einem vielfältigen Anforderungsprofil gerecht werden:

- zum einen sollen die resultierenden Komplexe löslich und möglichst monomer sein (bei ausschließlicher Verwendung von H-Atomen als Reste $R_c$ entstehen beispielsweise Decamere!)
- zum anderen müssen diese Komplexe auch stabil genug sein, um überhaupt unzersetzt zu sublimieren (Beseitigung des CH-bulks), woran die "harten" Donoratome entscheidenden Anteil haben
- und zum weiteren sollen diese Komplexe leicht genug sein, um bei möglichst niedrigen Temperaturen (weniger als 175 °C) zu sublimieren.

Das gleichzeitige Erfüllen aller drei Randbedingungen hat sich im Fall der Seltenerdmetalle als besonders schwierig erwiesen.

Die hier vorgestellten (CHDo)-Alkoxide zeichnen sich gegenüber den (CH)- und (CHX)-Alkoxiden dadurch aus, daß durch die Donorfunktionalitäten (Do) zusätzliche Koordinationsstellen besetzt werden und so Verhältnisse geschaffen werden, die denen bei der Verwendung von β-Diketonaten ähneln. Die (CHDo)-Alkohole ermöglichen jedoch gegenüber den β-Diketonaten eine größere Flexibilität in der Ligandensphäre. Erst durch das Zusammenwirken von Donorfunktionalität und sterischer Beanspruchung (die organischen Reste enthalten überwiegend oder ausschließlich Alkylgruppen mit bis zu vier Kohlenstoffatomen) werden die hervorragenden Eigenschaften der erfindungsgemäßen Alkoxide erreicht. Durch optimale Wahl der Kohlenstoffreste, der Donoratome Do und der Donorreste $R_{Do}$ kann gezielt Einfluß auf die sterische Sättigung und die Stabilität des Komplexes genommen werden. Aus diesen Überlegungen heraus kann auch ein optimaler Bereich für die Masse des Liganden abgeleitet werden. Eine hohe sterische Beanspruchung ergibt sich, wenn das Atomgewicht des Liganden über 90 liegt. Je nach Oxidationsstufe des Zentralmetalls ist auch eine höhere Untergrenze (100 bzw. 110) vorteilhaft. Andererseits läßt das Problem des CH-bulks ein Atomgewicht über 210 (in Spezialfällen bei verringerter Oxidationsstufe über 250) nicht mehr als günstig erscheinen.

Jeder Ligand bildet mit M einen Metalla-Cyclus, dessen Ringgröße nicht auf die Mindestzahl von fünf Atomen beschränkt ist, sondern auch mehr Atome umfassen kann, insbesondere sechs (oder auch sieben) Atome entsprechend folgender Struktur:

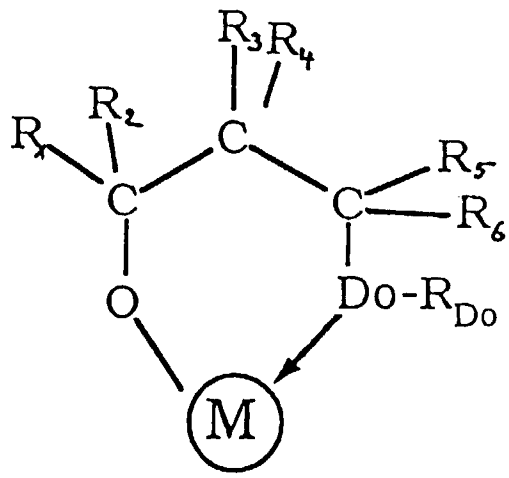

(2)

Die entsprechende Kurzschreibweise des Liganden lautet dann: O-$CR_1R_2$-$CR_3R_4$-$CR_5R_6$-$DOR_{Do}$.

Die hier vorgestellten Alkoxide und speziellen Alkohole sind sehr gut löslich in n-Hexan, Toluol und Ether in Übereinstimmung mit ihrer ausgezeichneten Eignung für den Sol-Gel-Prozeß.

Die erfindungsgemäßen Alkoxide der Seltenerdmetalle zeichnen sich durch die folgende Ligandenstruktur aus:

Die an Kohlenstoffatome gebundenen organischen Reste $R_c$ der Formel (1) bzw. (2), nämlich $R_1$ - $R_4$, bzw. auch $R_5$ und $R_6$ werden durch H, Me, Et, $^i$Pr oder $^t$Bu besetzt unter Ausschluß einer gleichzeitigen Besetzung aller vier bzw. sechs Reste durch H-Atome, während der organische Donorrest $R_{Do}$ durch ein oder mehrere der Gruppen Me, Et, $^i$Pr oder $^t$Bu besetzt wird.
Hierbei bedeuten

| | |
|---|---|
| H | = H-Atom |
| Me | = Methylgruppe $CH_3$ |
| Et | = Ethylgruppe $C_2H_5$ |
| $^i$Pr | = Isopropylgruppe $C_3H_7$ |
| $^t$Bu | = tert.-Butylgruppe $C_4H_9$ |

Bei gleichzeitiger Besetzung aller vier organischen Reste durch H-Atome wäre der Effekt der Oligomerisierung durch Brückenbindung zu stark ausgeprägt.

Die organischen Reste $R_c$ verschiedener an der Bildung des Alkoxid-Rings gemäß (1) oder (2) beteiligter Kohlenstoffatome können auch miteinander als Alkyliden-Ketten verbunden sein, so daß z.B. zwei dieser $R_c$-Reste eine Cyclohexyl- oder auch Cyclopentyl-Gruppe auf- bauen gemäß den folgenden Strukturen (3) und (4):

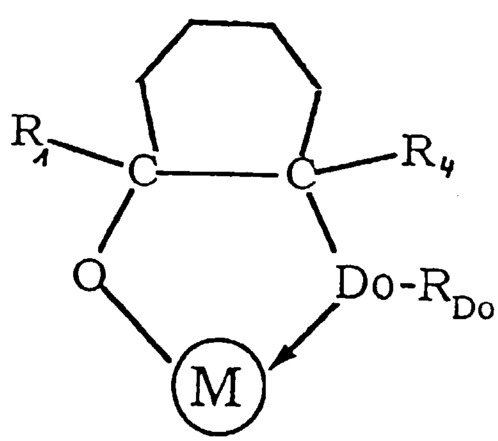

(3)

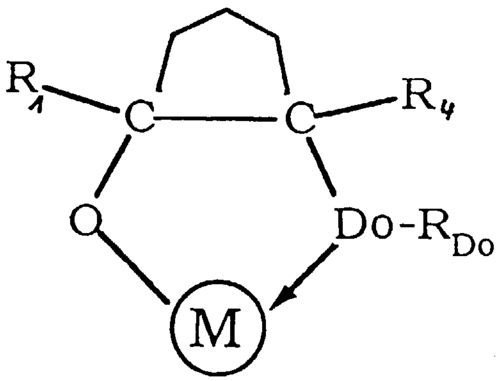

(4)

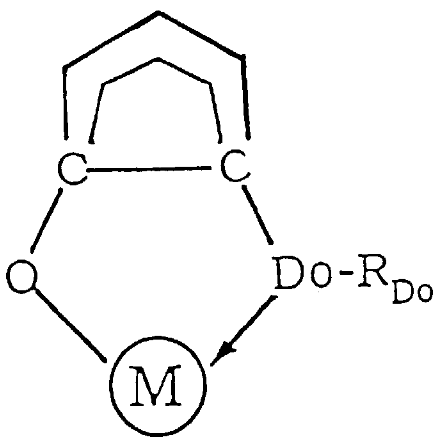

(4a)

Dabei können diese organischen Reste rein formal als $(-CH_2-)_n$-Gruppen aufgefaßt werden (n = ganze Zahl, bevorzugt ist n = 2...4). Die miteinander verbundenen organischen Reste bieten eine gute Möglichkeit der Einflußnahme auf die Löslichkeit der Alkoxide, wobei als cyclische Reste nicht nur H-Atome, sondern auch Alkyl-Reste wie Methyl oder Ethyl in Frage kommen. Insbesondere können zwischen zwei C-Atomen auch zwei Ringe ausgebildet sein (s. Struktur (4a)).

Zur Erhöhung der Koordinationszahl kann einer (oder zwei) der organischen Kohlenwasserstoff-Reste $R_c$ einen sog. Donorarm bilden, der eine weitere Donorfunktionalität enthält: $CR_aR_b$ - $DoR_{Do}$. Anders ausgedrückt bildet dieser Donorarm in bezug auf das Metallzentrum M einen zusätzlichen, mit dem ersten Metalla-Cyclus verknüpften Metalla-Cyclus. Er hat mit dem ersten Metalla-Cyclus zumindest das Fragment M-$OC_\alpha$ gemein, wobei die Vorschrift für seinen Aufbau - für sich allein betrachtet - wieder dem für den ersten Metalla-Cyclus entspricht. Damit läßt sich die Koordinationszahl des Seltenerdmetalls in seiner üblichen Oxidationsstufe (dreiwertig) von sechs auf neun erhöhen. Die Leistungsfähigkeit der Erfindung wird eindrucksvoll dadurch unterstrichen, daß sich die erfindungsgemäße Konzeption auch auf anomale Oxidationsstufen der Seltenerdmetalle (zweiwertig bzw. vierwertig) erweitern läßt. Insbesondere bei zweiwertigen Komplexen stellen zusätzliche Donorfunktionalitäten eine elegante Technik dar, um die Koordinationszahl von vier auf sechs oder sogar acht zu erhöhen. Bevorzugt werden dabei die organischen Reste des α-ständigen Kohlenstoffatoms des Alkoxid-Liganden mit einer (oder auch zwei) zusätzlichen Donorfunktionalitäten ausgestattet. Dabei sollte im Fall der Verwendung einer zweiten Donorfunktionalität diese aus sterischen Überlegungen heraus vorteilhaft einen verlängerten Ring (unter Einschluß des zentralen Metallatoms also einen Sechs- oder Siebenring) bilden. Möglich ist jedoch auch die Anbindung der Donorfunktionalität an das β-ständige Kohlenstoffatom. Dies gilt insbesondere für die zweite zusätzliche Donorfunktionalität, wobei u.U. auf einen verlängerten Ring verzichtet werden kann.

Bei der vierwertigen Oxidationsstufe kann auf zusätzliche Donorfunktionalitäten verzichtet werden. Vorteilhaft ist hier die Verwendung leichter Liganden, deren Atomgewichte bevorzugt zwischen 90 und 150 liegen. Höhere Atomgewichte sind jedoch nicht ausgeschlossen.

Als Donoratom für die "harten" Kationen der Seltenen Erden eignen sich entsprechend harte Donorfunktionalitäten, wie O oder N. Andere Atome, z.B. das weiche P, haben sich als ungeeignet erwiesen.

Die Vertreter der erfindungsgemäßen (CHDo)-Alkoxide zeigen eine niedrigere Molekülsymmetrie als die β-Diketonatkomplexe. Dies äußert sich in einer weniger dichten Packung im Kristallgitter und in einer möglichen höheren Flüchtigkeit. Besonders vorteilhaft können als organische Reste optisch aktive Gruppen eingebaut werden, welche sich erheblich einfacher realisieren lassen als im Fall der β-Diketonate. Bei Verwendung von Racematen der optischen Isomeren ergibt sich eine Schmelzpunktabsenkung gemäß dem bekannten Raoultschen Gesetz. Die optische Aktivität ist daher ein wichtiges zusätzliches Kriterium, um die Flüchtigkeit der Alkoxide durch geeignete Wahl der organischen Reste zu optimieren. Insbesondere eignen sich optisch aktive Kohlenstoffatome (C*) in der α-Position (siehe Tabelle 1, Alkohole 1a, 1b, 1c/d) und evtl. zusätzlich in der β-Position (siehe Tabelle 1, Alkohol 1i). Auch die cyclischen Verbindungen 1j-l aus Tabelle 1 sind optisch aktiv.

Cyclische Verbindungen haben zudem weitere Vorteile. Der CH-bulk ist weniger problematisch. Das Donoratom ist sterisch sehr gut fixiert, d.h. die Ringgeometrie ist schon durch den Liganden festgelegt. Die räumliche Struktur der cyclischen Verbindungen gestattet es, daß der Metallkern von den Alkoxy-Liganden ideal umschlossen ist.

Die hier vorgestellte Klasse von Liganden erlaubt es, für jedes Seltenerdmetall ein spezielles, maßgeschneidertes Ligandensystem zu schaffen. Beispielsweise hat sich für Neodym und Yttrium als Metallkern der Ligand $OC^iPr_2CH_2OEt$ als sehr vorteilhaft erwiesen. Der Sublimationspunkt (bei $10^{-3}$ mbar) des homoleptischen Alkoxids liegt bei nur 115 °C für Nd und 95 °C für Y als Metallkern.

Die Verbindungen sublimieren nahezu rückstandslos und unzersetzt, d.h. die analytischen Werte des Sublimates stimmen mit denen der Ausgangsverbindung überein, z.B. elementaranalytische Werte für

| $Y(OC^iPr_2CH_2OEt)_3$ | C | H |
|---|---|---|
| Theorie | 59.16 | 10.43 |
| vor der Sublimation | 58.86 | 10.52 |
| nach der Sublimation | 59.72 | 10.72 |

Ein weiteres wichtiges Kriterium für die Flüchtigkeit erhält man durch das Auftreten des Molekül-Peaks im Massenspektrum (siehe Fig. 1 und 2).

Im folgenden sollen mehrere Ausführungsbeispiele für Alkohole und daraus hergestellte Alkoxy-Liganden angegeben werden sowie das Herstellverfahren näher erläutert werden.

Figur 1 und 2 zeigen das Massenspektrum von $Nd(OC^tBu_2CH_2OEt)_3$ und $Y(OC^iPr_2CH_2OEt)_3$.

<u>Ausführungsbeispiele für Alkohole</u>

In Tabelle 1 sind Beispiele für besonders geeignete Alkohole aufgeführt. In Anlehnung an die daraus herstellbaren (CHDo)-Alkoxide sollen sie hier als (CHDo)-Alkohole bezeichnet werden.

Als besonders günstig für die Herstellung von Alkoxiden hat sich die Verwendung der neuen CHDo-Alkohole 1a-1d erwiesen, die gewissermaßen für die Herstellung von Seltenerdmetall-Komplexen ideale Voraussetzungen vorweisen, jedoch auch für die Synthese anderer Verbindungen eingesetzt werden können.

Die Charakterisierung der jeweiligen Verbindung erfolgte mit IR-Spektren, NMR- und Massenspektroskopie sowie elementaranalytisch. In diesem Zusammenhang ist es von besonderer Bedeutung, als organische Reste auch Phenylgruppen zu verwenden, da damit bestückte Verbindungen sehr leicht einer Röntgenstrukturanalyse zugänglich sind und daher Rückschlüsse auf die Struktur verwandter phenylfreier Verbindungen zulassen.

Tabelle 1

| Alkohol | Kurzbezeichnung | Summenformel | Atomgew. |
|---|---|---|---|
| $HOC^*H^tBuCH_2OEt$ | 1a | $C_8H_{18}O_2$ | 146 |
| $HOC^*H^tBuCH_2NEt_2$ | 1b | $C_{10}H_{23}NO$ | 173 |
| $HOC^tBu_2CH_2OEt$ | 1c | $C_{12}H_{26}O_2$ | 202 |
| $HOC^iPr_2CH_2OEt$ | 1d | $C_{10}H_{22}O_2$ | 174 |
| $HOC^{*i}Pr^tBuCH_2OEt$ | 1c/d | $C_{11}H_{24}O_2$ | 190 |
| $HOCEt_2CH_2OEt$ | 1e | $C_8H_{18}O_2$ | 146 |
| 2-methoxy-cyclopentanol | 1j | $C_7H_{14}O_2$ | 130 |
| 2-methoxy-cyclopentanol | 1k | $C_6H_{12}O_2$ | 116 |
| 2-Dimethylamino-cyclohexanol | 1l | $C_8H_{15}ON$ | 141 |

Herstellung der Alkohole

Die CHDo-Alkohole 1a - e lassen sich aus der entsprechenden Carbonylverbindung bzw. Epoxid leicht darstellen. Sie sind destillativ sehr gut zu reinigen und in guten Ausbeuten erhältlich. 1c erfordert aufgrund der enormen sterischen Beanspruchung lange Reaktionszeiten. Die Alkohole 1j - 1 werden ebenfalls aus Carbonylverbindungen erhalten.

Im einzelnen erfolgt die Herstellung der vier Alkohole 1a - 1d folgendermaßen, wobei die Synthese gaschromatographisch und massenspektrometrisch verfolgt wurde.

1) 1-ethoxy-3.3-dimethyl-2-butanol ($^t$Butylethoxymethylcarbinol, **1a**).

Ungefähr 70 ml (45 mmol) $ClMgCH_2OC_2H_5$-THF-Lösung werden bei -25 °C in einem kühlbaren Schlenkrohr vorgelegt. Dann werden bei -15 °C 4.0 ml (3.17 g, 36 mmol) Pivaldehyd, mit Ether ergänzt auf 20 ml, innerhalb von 30 min zugetropft. Für die später erfolgende Trennung der organischen und wässrigen Phase ist die Verwendung von Ether vorteilhaft, da sich THF sehr gut mit Wasser vermischt. Eine GCMS-Analyse zeigt nach 2 h schon vollständige Umsetzung an. Nach Erwärmen der Reaktionslösung von -15 °C auf -5 °C wird auf 50 g Eis gegossen. Danach wird so viel gesättigte $NH_4Cl$-Lösung zugegeben, bis sich gerade alles löst. Die organische Phase wird mit je 30 ml gesättigter $NaHCO_3$ und zweimal Eiswasser gewaschen und die vereinigten Wasserphasen nochmals mit Ether gewaschen. Danach werden die vereinigten organischen Phasen über $MgSO_4$ getrocknet. Fraktionierte Destillation liefert 1a in sehr guten Ausbeuten (88 °C/ 55 mbar, 92 %).

2) 1-diethylamino-3,3-dimethyl-2-butanol **(1b)**.

6.5 g (66 mmol) 3,3-Dimethyl-1,2-butanoxid werden zusammen mit einem Gemisch aus 20 ml $^t$BuOH und 5 ml $H_2O$ in einem 100 ml-Zweihalsrundkolben vorgelegt. Bei 0 °C werden 9 ml (123 mmol) Diethylamin zugetropft. Da bei dieser Temperatur noch keine Umsetzung erfolgt, wird die Reaktionslösung 24 h bei 60 °C gerührt. Die anschließende fraktionierte Destillation liefert den Alkohol in guten Ausbeuten (65 °C/ 15 mbar; 80 %).

3) 3-ethoxy-2.2.4.4-tetramethyl-3-pentanol (Di$^t$butylethoxymethylcarbinol, **1c)**.

Ungefähr 70 ml (44 mmol) $C1MgCH_2OC_2H_5$ (x-molare Lösung in THF) werden bei -25 °C in einem kühlbaren Schlenkrohr vorgelegt. Dann werden bei -15 °C 5.0 g (35 mmol) Di$^t$butylketon, mit Ether ergänzt auf 30 ml, sehr langsam zugetropft. Nach 4 h Reaktionszeit liegt das Produkt/Keton-Verhältnis bei 1:16.5.

Nach 93 h Rühren, währenddessen die Reaktionsmischung kontinuierlich auf -5 °C erwärmt wird, beträgt das Verhältnis 1.1:1. Die Reaktion wird durch Ausgießen auf 50 g Eis gestoppt. Es wird so viel gesättigte $NH_4Cl$-Lösung zugegeben, bis sich alles löst. Dann wird die organische Phase mit je 30 ml gesättigter $NaHCO_3$-Lösung und zweimal mit Eiswasser gewaschen. Die vereinigten Wasserphasen werden nochmals mit 50 ml Ether gewaschen und die vereinigten organischen Phasen über $MgSO_4$ getrocknet. Destillation bei 68 °C/ 4 mbar liefert 1c als farblose Flüssigkeit mit dem typischen "Tritox-H-Geruch" (50 %).

4) 3-ethoxy-2.4-dimethyl-3-pentanol (Di$^i$propylethoxymethylcarbinol, **1d)**.

In einem kühlbaren Schlenkrohr werden ungefähr 65 ml (60 mmol) $ClMgCH_2OC_2H5$ bei -20 °C vorgelegt. Bei -15 °C werden unter kräftigem Rühren 8.5 ml (6.8 g 59.5 mmol) Di$^i$propylketon, mit Ether ergänzt auf 35 ml, innerhalb 1 h zugetropft. Nach erfolgter Zugabe wird weiterhin 48 h bei dieser Temperatur gerührt, wobei ein weißer Niederschlag entsteht und das Keton vollständig umgesetzt wird. Innerhalb von 4 h läßt man die Reaktionsmischung auf -5 °C erwärmen und gießt dann auf 75 ml Eis. Es wird so viel gesättigte $NH_4Cl$-Lösung hinzugegeben, bis zwei klare Phasen vorliegen. Die organische Phase wird mit je 30 ml gesättigter $NaHCO_3$-Lösung und zweimal Eiswasser gewaschen. Die vereinigten Wasserphasen werden mit 50 ml Ether gewaschen und die vereinigten organischen Phasen über $MgSO_4$ getrocknet. Vorsichtige fraktionierte Destillation (Badtemp. max. 60 °C, sonst Dehydratisierung!) liefert **1d** in hohen Ausbeuten (53 °C/1 mbar, 95 %).

Ausführungsbeispiele für Alkoxide

In Tabelle 2 sind die Sublimationspunkte (bei $10^{-3}$mbar) von vier ausgewählten Alkoxiden angegeben. Als Seltenerdmetall ist repräsentativ Neodym bzw. Yttrium verwendet. Diese beiden Elemente überdecken hinsichtlich ihres Kationenradius das bei den Seltenerdmetallen vorhandene Spektrum. Bei Sechsfach-Koordination beträgt der effektive Kationenradius der Seltenerdmetalle zwischen (1,032 A (La) und 0,861 A (Lu)). Im Fall des Neodyms liegt er bei 0,983 A, für Yttrium bei 0,900 A.

In ähnlicher Weise lassen sich auch die anderen in Tab. 1 aufgeführten Alkohole in Alkoxide $(M(OR)_3$ umsetzen. Das Atomgewicht dieser Liganden unterscheidet sich um eine Einheit von dem der zugehörigen Alkohole.

Tabelle 2

| Alkoxid $M(OR)_3$ | Sublimationspunkt | Kurzbezeichnung |
|---|---|---|
| $Nd(OCH^tBuCH_2OEt)_3$ | 175 °C | 3a |
| $Nd(OCH^tBuCH_2NEt_2)_3$ | 150 °C | 3b |
| $Nd(OC^tBu_2CH_2OEt)_3$ | 125 °C | 3c |
| $Nd(OC^iPr_2CH_2OEt)_3$ | 115 °C | 3d |
| $Y(OC^iPr_2CH_2OEt)_3$ | 95 °C | 3e |

Diese Verbindungen können ohne Zersetzung sublimiert werden in Temperaturbereichen vergleichbar denen der β-Diketonat-Komplexe. Diese neuen Seltenerdmetall-Alkoxide müssen zudem den wenigen Vertretern chelatstabilisierter homoleptischer organometallischer Metallverbindungen zugeordnet werden.

Die hier beschriebenen Nd- und Y-Komplexe sind extrem luft- und feuchtigkeitsempfindlich. Sie müssen daher in einer Atmosphäre von Reinstargon und unter Einsatz der hierfür üblichen Arbeitstechnik (Schlenk-, Hochvakuum-, Glove-Box-Technik) behandelt werden.

Herstellung der Alkoxide der Oxidationsstufe III

Die Alkoxide werden nach der an sich bekannten "Silylamid"-Route gemäß Formel (5) hergestellt (Ln = Lanthanoid-Metallkern).

(5)

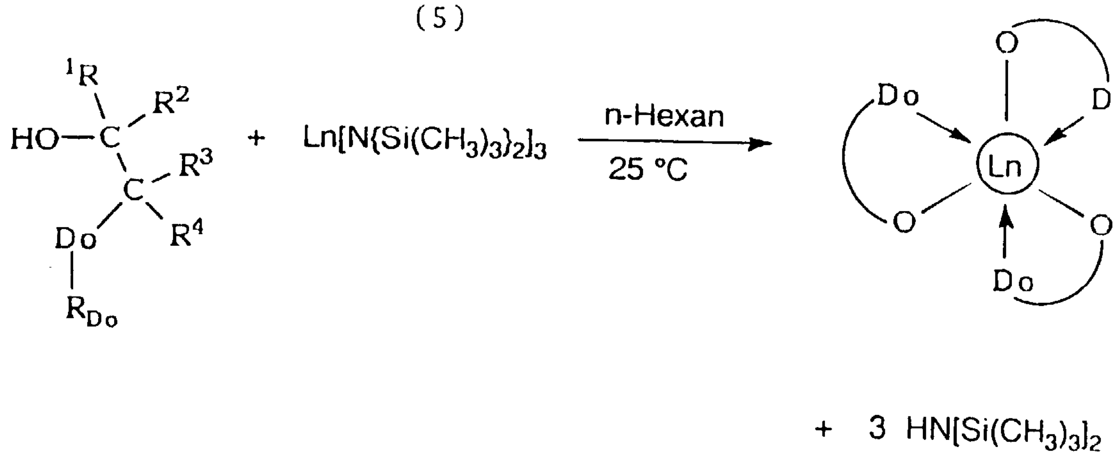


Beispielsweise erhält man durch Zugabe der Alkohole **1a-d** zu der hellblauen Suspension des Ln-Amids **2**, z. B. $Nd(N(Si(CH_3)_3)_2)_3$, in wenig Hexan klare blaugefärbte Lösungen in stark exothermer Reaktion. Nach Entfernen des Lösungsmittels bleiben blaue Öle von **3a-d** (mehrere Stereoisomere) zurück, die unterschiedlich schnell kristallisieren.

Im einzelnen erfolgt die Synthese folgendermaßen:

1. Tris(1-ethoxy-3.3-dimethyl-2-butoxy)neodym(III) **(3a)**. In einer Glove-Box werden 0.86 g (1.38 mmol) Nd-Amid **2a** (Ln = Nd) und 0,61 g (4.17 mmol) Alkohol **1a** zusammen in einen 100 ml-Rundkolben abgewogen. Bei Zugabe des Amids **2a** zum Alkohol **1a** erhält man eine tiefviolette Suspension unter stark exothermer Reaktion. Es werden sofort 30 ml Hexan in einer Hochvakuum-Apparatur aufkondensiert. Es geht fast alles in Lösung.

Anschließend wird 22 h bei Raumtemperatur gerührt. Von dem leichten samtigen weißen Niederschlag wird abfiltriert und das Lösungsmittel vom blauvioletten Filtrat im Vakuum (1 mbar) entfernt. Nach 2 h Trocknen bei $10^{-4}$ mbar erhält man **3a** als einen blauen hochviskosen Rückstand.

2. Tris(1-diethylamino-3.3-dimethyl-2-butoxy) neodym(III) **(3b)**. In einer Glove-Box werden 0.90 g (1.44 mmol) Nd-Amid **2a** in einen 100 ml-Rundkolben abgewogen. Es werden 30 ml frisch umkondensiertes Hexan dazupipettiert und 5 min gerührt. Zu dieser hellblauen Suspension werden 0.75 g (4.33 mmol) des Alkohols **1b** getropft. Die Suspension klart während der Zugabe zu einer blauvioletten Lösung auf. Nach 40 h Rühren bei Raumtemperatur

hat die Farbe nach blaßviolett umgeschlagen; es liegt immer noch eine klare Lösung vor. Das Hexan wird im Vakuum (1 mbar) entfernt und der hochviskose Rückstand 4 h im Vakuum ($10^{-3}$ mbar) getrocknet.

3. Tris(3-ethoxy-2.2,4.4-tetramethyl-3-pentoxy) neodym(III) **(3c)**. 0.81 g (1.30 mmol) Nd-Amid **2a** werden in einer Glove-Box in einen 100 ml-Rundkolben eingewogen. Mit einer Pipette werden 30 ml frisch umkondensiertes Hexan dazugegeben. Die hellblaue Suspension wird 5 min gerührt. Dann werden 0.79 g (3.90 mmol) Alkohol **1c** zugetropft. Während der Alkoholzugabe beobachtet man Aufklaren der Suspension zu einer blauvioletten Lösung. Nach 25 h Rühren bei Raumtemperatur ist die Lösung immer noch klar und die Farbe unverändert. Das Reaktionsgefäß wird aus der Glove-Box genommen und das Lösungsmittel im Vakuum (1 mbar) entfernt. Die Hexanphase enthält nur freies Amin. Der hellblaue, ölige Rückstand wird 2 h im Vakuum ($10^{-3}$ mbar) getrocknet (quantitative Umsetzung). Die Verbindung wird über Nacht in der Glove-Box fest und kann bei 125 °C/$10^{-3}$ mbar unzersetzt als hellblaue Verbindung **3c** sublimiert werden.

In Figur 1 ist ein Massenspektrum der Verbindung **3c** gezeigt. Klar erkennbar ist vor allem der Molekül-Peak bei 748, der zeigt, daß es sich bei **3c** tatsächlich um ein stabiles Monomer handelt. Die Peaks niedrigerer Masse rühren von Molekülfragmenten her (z.B. 690 = M. - $C_4H_9$).

4. Tris(3-ethoxy-2.4-dimethyl-3-pentoxy) neodym(III) **(3d)**. In einer Glove-Box werden 0.53 g (0.85 mmol) des Nd-Amids **2a** (Ln = Nd) in einen 50 ml-Rundkolben abgewogen. Frisch vakuumtransferiertes n-Hexan wird in der Glove-Box auf -35 °C gekühlt und dann zu **2a** gegeben (25 ml). Zu der hellblauen Suspension wird vorsichtig 0.47 g (2.67 mmol) des Alkohols 1d getropft. Die Reaktionsmischung klart sich schon während des Zutropfens vollständig zu einer blauvioletten Lösung auf. Die Lösung wird 24 h bei Raumtemperatur gerührt. Danach wird das Lösungsmittel im Vakuum entfernt (1 mbar) und der ölige Rückstand noch 4 h im Hochvakuum ($10^{-3}$ mbar) getrocknet. Die Hexanphase enthält nur freies Amin. **3d** kann ab 115 °C/ $10^{-3}$ mbar rückstandslos und unzersetzt sublimiert werden.

5. Tris(3-ethoxy-2.4-dimethyl-3-pentoxy) yttrium(III) **(3e)**. In einer Glove-Box werden zu 1.03 g (1.81 mmol) des Y-Amids **2b** (Ln = Y) suspendiert in 30 ml n-Hexan (frisch vakuumtransferiert, -35 °C) 0.94 g (5.37 mmol) Alkohol 1d getropft. Die resultierende farblose Lösung wird 40 h bei Raumtemperatur gerührt. Danach wird das Lösungsmittel im Vakuum (1 mbar) entfernt und der hochviskose Rückstand im Hochvakuum ($10^{-3}$ mbar) 5 h getrocknet. Dieser kristallisiert über Nacht analysenrein aus und kann ab 95 °C/ $10^{-3}$ mbar sublimiert werden.

In Figur 2 ist ein Massenspektrum der Verbindung **3e** dargestellt. Charakteristisch ist der Molekül-Peak bei 609, der wieder die Existenz des stabilen Monomers beweist. Das wichtigste Molekülfragment liegt bei 565 (= M. - $C_3H_7$).

Die Alkoxide gemäß der Erfindung eignen sich aufgrund ihrer exzellenten Löslichkeit in Ether, THF, n-Hexan und Toluol für den Sol-Gel-Prozeß und aufgrund ihrer niedrigen Sublimationstemperaturen für die MOCVD-Technik. Dadurch lassen sich extrem reine Schichten herstellen.

Ein Anwendungsgebiet ist die Verwendung als III-Komponente bei den "I, II, III-Supraleitern", die aus dünnen Schichten supraleitenden Materials bestehen, die auf ein keramisches Substrat mittels MOCVD aufgetragen wird (Appl. Organometallic Chem. 1990, S. 439-449). Ein Beispiel ist der $YBa_2Cu_3O_{7-\delta}$ -Supraleiter.

Ein weiteres Anwendungsgebiet ist die Herstellung dünner Oxidschichten auf metallischen Elektroden zur Förderung der Elektronenemission. Für diese Anwendung besonders geeignet ist $La_2O_3$ wegen der geringen Elektronenaustrittsarbeit. Beispielsweise wird es auf massive Wolfram-Elektroden für druckentladungslampen aufgetragen. Insbesondere handelt es sich dabei um Kurzbogenlampen mit Quecksilberdampf- bzw. Xenongasfüllung, bei denen eine hohe Bogenstabilität und hohe Konstanz der Elektronenemission wesentlich ist. Der besondere Vorteil dieser Art des Aufbringens eines Emitters ($La_2O_3$) aus der Gasphase ist, daß er auf der Elektrode in räumlicher Vorzugsrichtung orientiert werden kann, so daß die Effektivität des Emitters erhöht wird. Ein anderes Anwendungsgebiet sind Metallhalogenid-Entladungslampen, bei denen lediglich der Schaft der Elektrode mit dem Seltenerdmetall-Oxid beschichtet ist, ähnlich der DE-OS 38 12 084. Hierfür eignen sich insbesondere Oxide von Y und La. Das dort angesprochene MOCVD-Verfahren für die Beschichtung beruht offensichtlich auf der Verwendung von β-Diketonaten.

Die erfindungsgemäßen Seltenerdmetall-Alkoxide lassen sich besonders vorteilhaft in einem MOCVD-Verfahren ohne zusätzliches reaktives Gas, d.h. sauerstoffhaltiges Gas, als Oxide abscheiden.

Insbesondere wird dabei ein niedriger Druck angewendet. Die Abscheidung erfolgt dabei durch thermische Zersetzung (bei ca. 400-500 °C) im Vakuum. Die Sublimationstemperatur des flüchtigen Alkoxids kann dabei unter 150 °C bleiben.

6. Herstellung der Alkoxide der Oxidationsstufe IV Besonderes Interesse verdienen hierbei Cer-Verbindungen. Lösliche Ce(IV)-Alkoxide, $Ce(OR)_4$ lassen sich als Precursor-Verbindung für $CeO_2$-Keramiken (Anwendung in

Katalyse, optische Additive, Ionenleitung, Gegenelektroden für elektrochrome Geräte) einsetzen. So werden seit geraumer Zeit dünne Filme aus $CeO_2$ als isolierendes und chemisch resistentes Material für Silizium Wafer untersucht. Diese Kombination ("buffer layer") eignet sich besonders gut als Unterlage für das Wachstum von Hochtemperatursupraleitern. Alternativ werden wiederum β-diketonato-Verbindungen diskutiert.

Bedingt durch die Vierwertigkeit des Cers werden zur Ausbildung einer homoleptischen Verbindung vier Alkohol-Liganden benötigt. Folglich bieten sich dem Metallzentrum in $Ce(OR)_4$ jetzt acht Koordinationsstellen. Dies entspricht dem folgenden Aufbau:

$Ce(OR)_4$ =

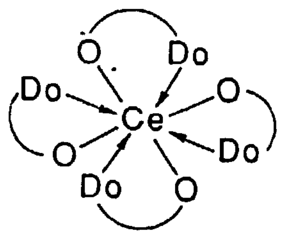

und

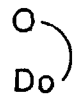

=

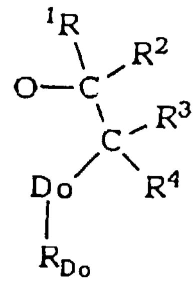

Folgende Alkoxide A und B zeigen eine besondere Eignung als Liganden für dieses System:

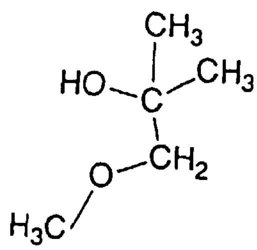

A

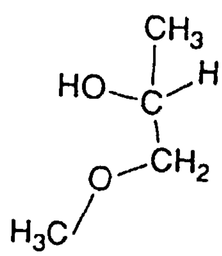

B

Syntheserouten:

$$CAN + 4\ ROH + 4\ NH_3 \rightarrow Ce(OR)_4 + 6\ NH_4NO_3$$

$$CAN + 6\ NaOR \rightarrow Ce(OR)_4 + 2\ NH_3 + 6\ NaNO_3 + 2\ ROH$$

(CAN = Ceramoniumnitrat, $(NH_4)_2Ce(NO_3)_6$)

Der Ionenradius von Cer(IV) beträgt bei 8-Koordination 0.97 A und ist damit etwas kleiner als der von Cer(III) bei 6-Koordination (1.01 A). Das heißt jetzt, daß in der Oxidationsstufe vier der sterische Anspruch in den CHDo-Alkoholen nicht mehr so groß sein muß wie bei den oben diskutierten $M(OR)_3$-Systemen. Dementsprechend sind daher CHDo-Alkohole mit kleinerem Molekulargewicht (Beispiel A: m = 104 und Beispiel B: m = 90) vorteilhaft.

7. Herstellung der Alkoxide der Oxidationsstufe II Besonderes Interesse verdienen hierbei Verbindungen von Samarium, Europium, Ytterbium oder Thulium. Alkoxide zweiwertiger Lanthanoide sind in der Literatur spärlich beschrieben. Es existiert erst ein strukturchemisch charakterisiertes Derivat, nämlich das Aryloxid-System Yb

$(OAr)_2L_x$ (OAr = $C_6H_2{}^tBu_2$-2,6-Me-4, L = thf, $OEt_2$, x = 2,3). Zweiwertige Seltenerd-Alkoxide $M(OR)_2$, als Vorstufe für M(II)-Oxide, MO, sind neue Verbindungen, die erst durch die Verwendung funktionalisierter Alkohole realisierbar werden.

Die magnetischen Eigenschaften dieser "reduzierten Oxide" (insbesondere SmO (goldgelb glänzend), EuO (dunkelrot) und YbO (grünlich-weiß)) machen sie, wie die entsprechenden homologen Monochalkogenide interessant für die Verwendung in elektronischen Speichereinheiten (z.B. Ferromagnetismus von EuO bei tiefen Temperaturen). Die Systeme MO sind nur unter extremen Bedingungen erhältlich (hohe Temperaturen, hohe Drucke). Ein gangbarer Weg zur Herstellung dieser "Zwangsoxide" ist die plasmachemische Abscheidung von flüchtigen zweiwertigen Vorstufen wie $M(OR)_2$. Auch bietet sich hier eine Abscheidung der oben diskutierten $M(OR)_3$-Vorstufen unter reduzierenden Bedingungen an (z.B. $H_2$-Plasma).

Im System $M(OR)_2$ stehen dem Metallzentrum nur zwei Liganden zur Verfügung, um seine Koordinationssphäre abzusättigen. Bei Verwendung der oben angeführten CHDo-Alkohole entspräche dies, bei Anstreben eines monomeren Alkoxid-Komplexes, einer Koordinationszahl von vier. Zweiwertige Lanthanoide sind um ca. 0.2 A größer als die entsprechenden dreiwertigen, z.B. ist der effektive Ionenradius von Eu(II) 1.17 A, der von Eu(III) 0.947 A bei einer Koordinationszahl von sechs.

Die CHDo-Alkohole lassen sich entsprechend modifizieren und dem "neuen Metallzentrum" anpassen. Um mindestens eine 6-Koordination zu gewährleisten, müssen insgesamt zwei Donorfunktionalitäten in einen Liganden eingebaut werden. Dies geschieht vorteilhaft mit Hilfe des Ersatzes des organischen Restes $R_1$ am ersten Kohlenstoffatom durch einen Donorarm $CR_aR_b$-$DoR_{Do}$.

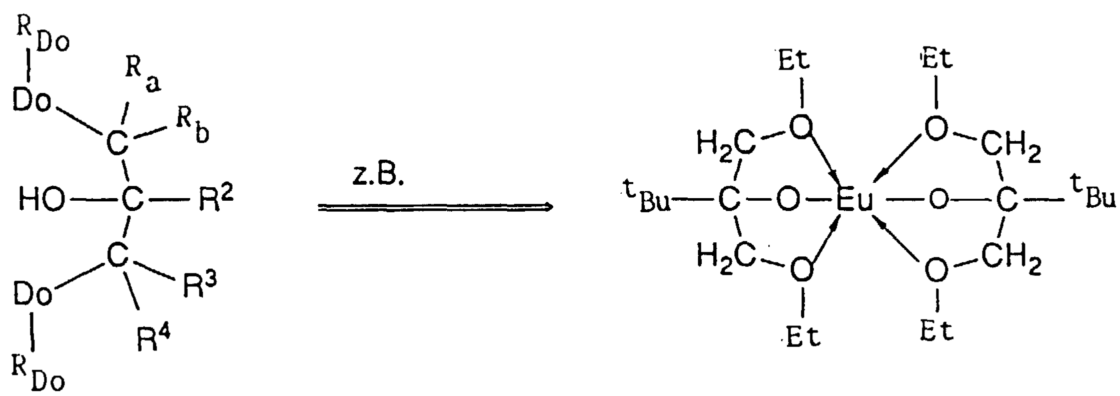


Das Metallzentrum ist nun gleichsam Teil von zwei Doppelringen, die sich schematisch auch als vier Fünfringe auffassen lassen, von denen jeweils zwei miteinander verknüpft sind; denkbar sind auch entsprechende Konfigurationen mit Sechsringen.

Dieses Prinzip läßt sich sogar dahingehend verallgemeinern, daß auch der zweite organische Rest des ersten Kohlenstoffatoms (α - C) oder ein Rest des zweiten Kohlenstoffatoms (β - C) mit einer Donorfunktionalität ausgestattet wird. Dabei bietet der Austausch eines weiteren organischen Restes durch einen weiteren Donorarm dem Metallzentrum zwei weitere Koordinationsstellen, womit letztendlich eine 8-Koordination erreicht werden kann. Aus sterischen Überlegungen sollte dieser zusätzliche Arm - zumindest im Fall seiner Ankopplung an das α - C - mindestens einen Sechsring, besser einen Siebenring, mit dem Metallzentrum bilden.

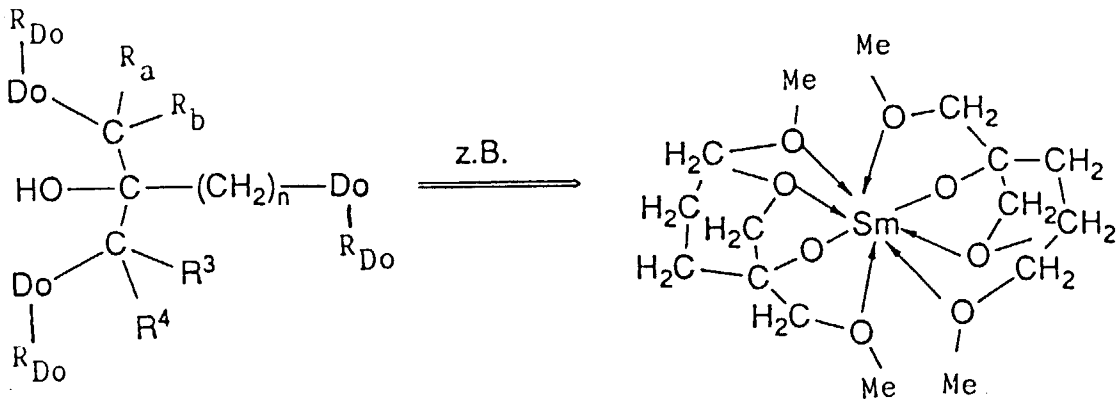


Syntheserouten:

$$LnI_2 + 2\,NaN(SiMe_3)_2 \xrightarrow{THF} Ln[N(SiMe_3)_2]_2(THF)_2 + 2\,NaI$$

$$Ln + Hg[N(SiMe_3)_2]_2 \xrightarrow{THF} Ln[N(SiMe_3)_2]_2(THF)_2 + Hg$$

$$Ln[N(SiMe_3)_2]_2(THF)_2 + 2\,ROH \xrightarrow[-\,THF]{n\text{-}Pentan} Ln(OR)_2 + 2\,HN(SiMe_3)_2$$

**Patentansprüche**

**1.** Seltenerdmetall-Alkoxide hoher Löslichkeit und Flüchtigkeit, gekennzeichnet durch Liganden (OR), die Donorfunktionalitäten mit einem Donoratom (Do) enthalten, und - unter Einschluß des zentralen Seltenerdmetallatoms (M) für den Liganden - einen mindestens fünfteiligen Ring bilden, der das Metallatom (M), ein benachbartes Sauerstoffatom (O), das keinen Substituenten besitzt, mindestens zwei (C) Kohlenstoffatome, die im folgenden als erstes und zweites Kohlenstoffatom bezeichnet sind, und ein Donoratom (Do) umfaßt,

wobei das dem substituentenfreien Sauerstoffatom direkt benachbarte C-Atom (also das α-ständige C-Atom) im folgenden als Cα-Atom bezeichnet ist;

mit jeweils zwei organischen Resten ($R_c$) pro Kohlenstoffatom, wobei die organischen Reste des ersten Kohlenstoffatoms mit $R_1$ und $R_2$ und die des zweiten Kohlenstoffatoms mit $R_3$ und $R_4$ bezeichnet werden, und mit organischen Resten $R_{Do}$ am Donoratom (Do);

wobei das Atomgewicht des einzelnen Liganden mindestens 90 beträgt und

wobei die organischen Reste ($R_c$) an den Kohlenstoffatomen (C) entsprechend einer der folgenden Möglichkeiten besetzt sind:

a) im Falle eines fünfteiligen Rings:

- durch H-Atome (H), Methylgruppen (Me), Ethylgruppen (Et), Isopropylgruppen ($^iPr$) oder tert.-Butylgruppen ($^tBu$), unter Ausschluß einer gleichzeitigen Besetzung aller Reste ($R_c$) durch H-Atome, wobei

die organischen Reste $R_3$ und $R_4$ jeweils H-Atome sind, und wobei zumindest einer der beiden organischen Reste $R_1$ oder $R_2$ durch die Gruppe Et oder eine Gruppe mit höherem Atomgewicht gebildet wird,

b) im Falle eines Rings mit mehr als fünf Teilen:

- durch H-Atome (H), Methylgruppen (Me), Ethylgruppen (Et), Isopropylgruppen ($^iPr$) oder tert.-Butylgruppen ($^tBu$), unter Ausschluß einer gleichzeitigen Besetzung aller Reste ($R_c$) durch H-Atome, wobei die organischen Reste $R_1$ oder $R_2$ nicht jeweils H-Atome sind,

c) im Falle eines mindestens fünfteiligen Rings:

- durch Alkyliden-Ketten, insbesondere $(-CH_2-)_n$-Gruppen (n = ganze Zahl), wobei organische Reste verschiedener Kohlenstoffatome durch Bildung von cyclischen Ringen miteinander verbunden sind,
- oder bis zu zwei der organischen Reste $R_c$ eine weitere Donorfunktionalität enthalten (im folgenden "Donor-Arme" genannt);

wobei das jeweilige Donoratom (Do) entweder O oder N ist und

wobei die organischen Reste ($R_{Do}$) am jeweiligen Donoratom durch ein oder zwei Gruppen, ausgewählt aus Me, Et, $^iPr$ oder $^tBu$, besetzt sind.

**2.** Seltenerdmetall-Alkoxide nach Anspruch 1, gekennzeichnet durch einen homoleptischen Aufbau entsprechend der Strukturformel $M(OR)_x$, mit x = 2 bis 4, wobei ein einziges Seltenerdmetall M von x gleichen Liganden OR umgeben ist.

**3.** Seltenerdmetall-Alkoxide nach Anspruch 2, dadurch gekennzeichnet, daß x = 3.

**4.** Seltenerdmetall-Alkoxide nach Anspruch 3, dadurch gekennzeichnet, daß das Atomgewicht eines Liganden 100-210 beträgt.

**5.** Seltenerdmetall-Alkoxide nach Anspruch 2, dadurch gekennzeichnet, daß x = 4.

**6.** Seltenerdmetall-Alkoxide nach Anspruch 5, dadurch gekennzeichnet, daß das Atomgewicht eines Liganden 90-210 beträgt.

**7.** Seltenerdmetall-Alkoxide nach Anspruch 2, dadurch gekennzeichnet, daß x = 2.

**8.** Seltenerdmetall-Alkoxide nach Anspruch 7, dadurch gekennzeichnet, daß das Atomgewicht eines Liganden 110-250 beträgt.

**9.** Seltenerdmetall-Alkoxide nach einem der vorhergehenden Ansprüche, gekennzeichnet durch einen oder mehrere Liganden, die jeweils mit dem Metallatom (M) einen fünfteiligen Ring bilden, mit folgendem Aufbau:

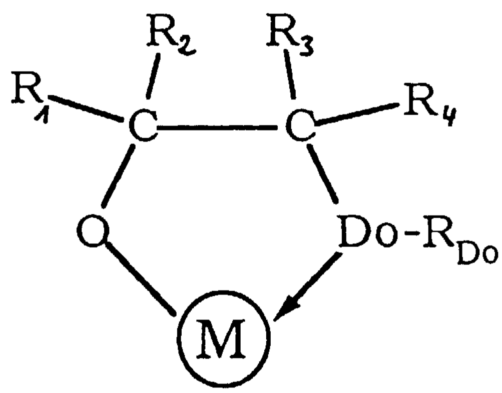

**10.** Seltenerdmetall-Alkoxide nach einem der vorhergehenden Ansprüche, gekennzeichnet durch einen oder mehrere Liganden, die jeweils mit dem Metallatom (M) einen sechsteiligen Ring bilden mit folgendem Aufbau:

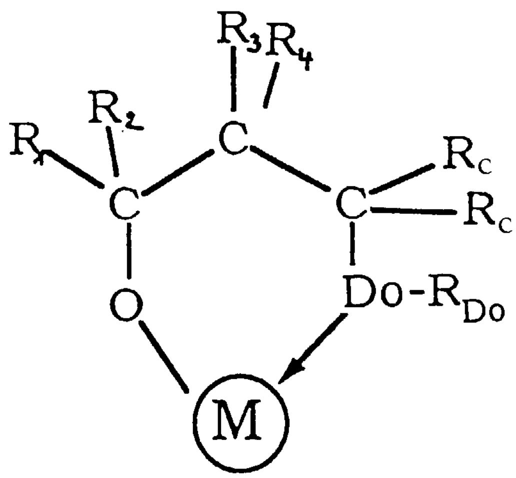

**11.** Seltenerdmetall-Alkoxide nach Anspruch 9, dadurch gekennzeichnet, daß die organischen Reste $R_3$ und $R_4$ jeweils H-Atome sind, wobei zumindest einer der beiden organischen Reste $R_1$ oder $R_2$ durch die Gruppe Et oder eine Gruppe mit höherem Atomgewicht gebildet wird.

**12.** Seltenerdmetall-Alkoxide nach Anspruch 1, dadurch gekennzeichnet, daß zumindest eines der am Alkoxid-Ring beteiligten Kohlenstoffatome optisch aktiv ist.

**13.** Seltenerdmetall-Alkoxide nach Anspruch 1, dadurch gekennzeichnet, daß im Fall des Vorhandenseins eines oder mehrerer cyclischer Ringe der Teil der organischen $R_c$-Reste, der miteinander verbunden ist, einen fünf- oder sechsteiligen Ring bildet.

**14.** Seltenerdmetall-Alkoxide nach Anspruch 3 oder 7, dadurch gekennzeichnet, daß zur Erhöhung der Koordinationszahl zumindest einer der organischen Reste $R_c$ als "Donorarm" mit folgendem Aufbau ausgeführt ist: $CR_aR_b$-Do-$R_{Do}$, unter Verwendung der Nomenklatur von Anspruch 1, wobei $R_a$ und $R_b$ donorfreie organische Reste entsprechend Anspruch 1 sind.

**15.** Seltenerdmetall-Alkoxide nach Anspruch 14,
dadurch gekennzeichnet, daß zur zusätzlichen Erhöhung der Koordinationszahl ein weiterer organischer Rest $R_c$ als "Donorarm" mit folgendem Aufbau ausgeführt ist:
- $(CH_2)_n$-Do-$R_{Do}$, wobei $n \geq 2$.

**16.** Alkohole zum Synthetisieren von flüchtigen Verbindungen, insbesondere von Alkoxiden, entsprechend einer der

folgenden Strukturformeln:
$HOC^tBU_2CH_2OEt$
$HOC^iPr_2CH_2OEt$.

**17.** Verfahren zur Herstellung von dünnen Schichten aus Oxiden der Seltenerdmetallen, gekennzeichnet durch ein MOCVD-Verfahren ohne Reaktans unter Verwendung flüchtiger Seltenerdmetall-Alkoxide gemäß Anspruch 1 bis 15.

## Claims

**1.** Rare earth metal alkoxides of high solubility and volatility, characterized by ligands (OR) which contain donor functionalities having a donor atom (Do) and, with inclusion of the central rare earth metal atom (M) for the ligand, form an at least five-membered ring which comprises the metal atom (M), an adjacent oxygen atom (O) which has no substituent, at least two carbon atoms (C), which are hereinafter referred to as first and second carbon atom, and a donor atom (Do),

where the carbon atom directly adjacent to the substituent-free oxygen atom (i.e. the $\alpha$-carbon atom) is hereinafter referred to as the $C\alpha$ atom;

having in each case two organic radicals ($R_c$) per carbon atom, where the organic radicals of the first carbon atom are designated as $R_1$ and $R_2$ and those of the second carbon atom are designated as $R_3$ and $R_4$, and having organic radicals $R_{Do}$ on the donor atom (Do);

where the atomic weight of the individual ligand is at least 90 and

where the organic radicals ($R_c$) on the carbon atoms (C) are occupied according to one of the following possibilities:

a) in the case of a five-membered ring:

- by H atoms (H), methyl groups (Me), ethyl groups (Et), isopropyl groups ($^i$Pr) or tert-butyl groups ($^t$Bu), with exclusion of a simultaneous occupation of all radicals ($R_c$) by H atoms, where the organic radicals $R_3$ and $R_4$ are each H atoms and where at least one of the two organic radicals $R_1$ or $R_2$ is the group Et or a group having a higher atomic weight,

b) in the case of a ring having more than five ring members:

- by H atoms (H), methyl groups (Me), ethyl groups (Et), isopropyl groups ($^i$Pr) or tert-butyl groups ($^t$Bu), with exclusion of a simultaneous occupation of all radicals ($R_c$) by H atoms, where the organic radicals $R_1$ or $R_2$ are not each H atoms,

c) in the case of an at least five-membered ring:

- by alkylidene chains, in particular $(-CH_2-)_n$ groups (n = an integer), where organic radicals having different numbers of carbon atoms are connected to one another to form cyclic rings,

- or up two of the organic radicals $R_c$ contain a further donor functionality (hereinafter referred to as "donor arms");

where the respective donor atom (Do) is either O or N and

where the organic radicals ($R_{Do}$) on the respective donor atom are occupied by one or two groups selected from among Me, Et, $^i$Pr, or $^t$Bu.

**2.** Rare earth metal alkoxides according to Claim 1, characterized by a homoleptic structure corresponding to the structural formula $M(OR)_x$, where x = 2 to 4, where a single rare earth metal M is surrounded by x identical ligands OR.

**3.** Rare earth metal alkoxides according to Claim 2, characterized in that x = 3.

**4.** Rare earth metal alkoxides according to Claim 3, characterized in that the atomic weight of a ligand is 100-210.

**5.** Rare earth metal alkoxides according to Claim 2, characterized in that x = 4.

**6.** Rare earth metal alkoxides according to Claim 5, characterized in that the atomic weight of a ligand is 90-210.

**7.** Rare earth metal alkoxides according to Claim 2, characterized in that x = 2.

**8.** Rare earth metal alkoxides according to Claim 7, characterized in that the atomic weight of a ligand is 110-250.

**9.** Rare earth metal alkoxides according to any of the preceding claims, characterized by one or more ligands each of which, together with the metal atom (M), form a five-membered ring having the following structure:

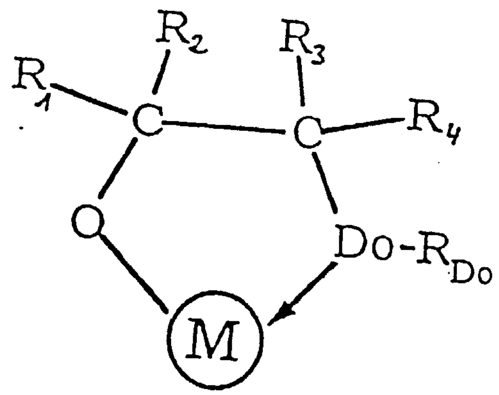

**10.** Rare earth metal alkoxides according to any of the preceding claims, characterized by one or more ligands each of which, together with the metal atom (M), form a six-membered ring having the following structure:

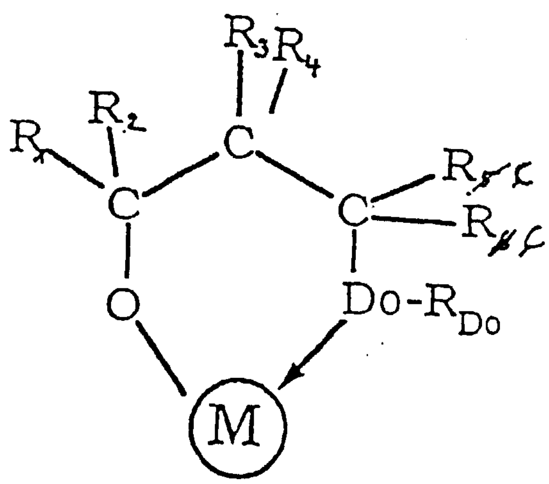

**11.** Rare earth metal alkoxides according to Claim 9, characterized in that the organic radicals $R_3$ and $R_4$ are each H atoms, where at least one of the two organic radicals $R_1$ or $R_2$ is the group Et or a group having a higher atomic weight.

**12.** Rare earth metal alkoxides according to Claim 1, characterized in that at least one of the carbon atoms in the alkoxide ring is optically active.

**13.** Rare earth metal alkoxides according to Claim 1, characterized in that if one or more cyclic rings are present, the

parts of the organic $R_c$ radicals which are connected to one another form a five- or six-membered ring.

**14.** Rare earth metal alkoxides according to Claim 3 or 7, characterized in that, to increase the coordination number, at least one of the organic radicals $R_c$ is a donor arm and has the following structure:
$CR_aR_b$-Do-$R_{Do}$, using the nomenclature of Claim 1, where $R_a$ and $R_b$ are donor-free organic radicals corresponding to Claim 1.

**15.** Rare earth metal alkoxides according to Claim 14, characterized in that, to additionally increase the coordination number, a further organic radical $R_c$ is a "donor arm" and has the following structure:
$-(CH_2)_n$-Do-$R_{Do}$, where $n \geq 2$.

**16.** Alcohols for the synthesis of volatile compounds, in particular of alkoxides, corresponding to one of the following structural formulae:
$HOC^tBU_2CH_2OEt$
$HOC^iPr_2CH_2OEt$.

**17.** Process for producing thin layers of oxides of rare earth metals, characterized by an MOCVD process without reagent using volatile rare earth metal alkoxides according to any of Claims 1 to 15.

**Revendications**

**1.** Alcoolates de métaux de terre rare, de grande solubilité et de grande volatilité, caractérisés par des ligands (OR) qui comportent des fonctionnalités de donneur par un atome donneur (Do) et qui forment, en incluant l'atome de terre rare (M) central pour le ligand, un cycle à au moins cinq chaînons qui englobe l'atome de métal (M), un atome d'oxygène (O) voisin qui n'a pas de substituant, au moins deux atomes de carbone (C) désignés dans ce qui suit comme étant le premier et le deuxième atome de carbone, et un atome donneur (Do),

l'atome de C immédiatement adjacent à l'atome d'oxygène sans substituant (donc l'atome de C en position $\alpha$) étant désigné dans ce qui suit comme étant l'atome $C\alpha$ ;
comprenant deux radicaux ($R_c$) organiques par atome de carbone, les radicaux organiques du premier atome de carbone étant désignés par $R_1$ et $R_2$ et ceux du deuxième atome de carbone par $R_3$ et $R_4$, et ayant des radicaux organiques $R_{Do}$ sur l'atome donneur (Do) ;
le poids atomique du ligand étant d'au moins 90 et
les radicaux ($R_c$) organiques étant placés sur les atomes (C) de carbone conformément à l'une des possibilités suivantes :

a) dans le cas d'un cycle à cinq chaînons :

- ce sont des atomes d'H (H), des groupes méthyle (Me), des groupes éthyle (Et), des groupes isopropyle ($^iPr$) ou des groupes butyle tertiaires ($^tBu$), étant exclu que tous les radicaux ($R_c$) soient des atomes d'H, les radicaux $R_3$ et $R_4$ organiques étant chacun des atomes d'H, et au moins l'un des deux radicaux $R_1$ ou $R_2$ organiques étant formé par le groupe Et ou un groupe ayant un poids atomique plus grand,

b) dans le cas d'un cycle ayant plus de cinq chaînons :

- ce sont des atomes d'H (H), des groupes méthyle (Me), des groupes éthyle (Et), des groupes isopropyle ($^iPr$) ou des groupes butyle tertiaires ($^tBu$), étant exclu que tous les radicaux ($R_c$) soient des atomes d'H, les radicaux $R_1$ ou $R_2$ organiques n'étant pas respectivement des atomes d'H,

c) dans le cas d'un cycle à au moins cinq chaînons :

- ce sont des chaînes alcoylidène, notamment des groupes $(-CH_2-)_n$ (n étant un nombre entier), les radicaux organiques des divers atomes de carbone étant reliés entre eux par formation de noyaux cycliques,
- ou jusqu'à deux des radicaux $R_c$ organiques comportent une autre fonctionnalité de donneur (désignés dans ce qui suit par "bras donneur") ;

l'atome donneur (Do) étant O ou N et
les radicaux ($R_{Do}$) organiques sur l'atome donneur étant formés par un ou deux groupes, choisis parmi Me, Et, $^{i}$Pr ou $^{t}$Bu.

2. Alcoolates de métaux de terre rare suivant la revendication 1, caractérisés par une structure homoleptique correspondant à la formule structurale $M(OR)_x$, x = 2 à 4, un métal de terre rare M unique étant entouré de x ligands OR identiques.

3. Alcoolates de métaux de terre rare suivant la revendication 2, caractérisés en ce que x est égal à 3.

4. Alcoolates de métaux de terre rare suivant la revendication 3, caractérisés en ce que le poids atomique d'un ligand est compris entre 100 et 210.

5. Alcoolates de métaux de terre rare suivant la revendication 2, caractérisés en ce que x = 4.

6. Alcoolates de métaux de terre rare suivant la revendication 5, caractérisés en ce que le poids atomique d'un ligand est compris entre 90 et 210.

7. Alcoolates de métaux de terre rare suivant la revendication 2, caractérisés en ce que x est égal à 2.

8. Alcoolates de métaux de terre rare suivant la revendication 7, caractérisés en ce que le poids atomique d'un ligand est compris entre 110 et 250.

9. Alcoolates de métaux de terre rare suivant l'une des revendications précédentes, caractérisés par un ligand ou par plusieurs ligands qui forment avec l'atome de métal (M) un cycle à cinq chaînons ayant la structure suivante :

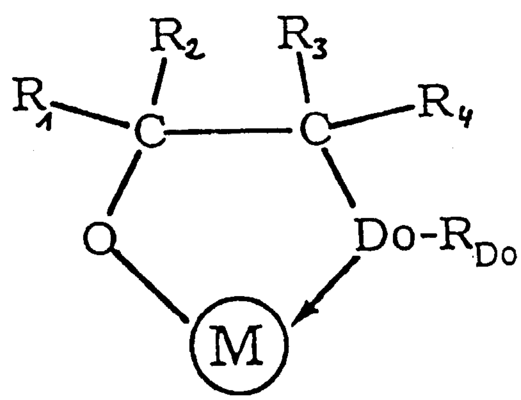

10. Alcoolates de métaux de terre rare suivant l'une des revendications précédentes, caractérisés par un ligand ou par plusieurs ligands qui forment respectivement avec l'atome de métal (M) un cycle à six chaînons ayant la structure suivante :

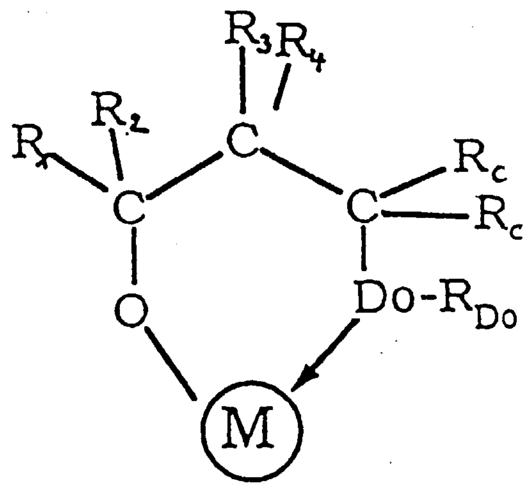

**11.** Alcoolates de métaux de terre rare suivant la revendication 9, caractérisés en ce que les radicaux organiques $R_3$ et $R_4$ sont respectivement des atomes d'H, au moins l'un des deux radicaux $R_1$ ou $R_2$ organiques étant formés par le groupe Et ou par un groupe ayant un poids atomique plus grand.

**12.** Alcoolates de métaux de terre rare suivant la revendication 1, caractérisés en ce qu'au moins un des atomes de carbone faisant partie du cycle alcoolate est actif optiquement.

**13.** Alcoolates de métaux de terre rare suivant la revendication 1, caractérisés en ce que si un noyau cyclique ou si plusieurs noyaux cycliques sont présents, la partie des radicaux $R_c$ organiques qui sont reliés entre eux forme un cycle à cinq chaînons ou à six chaînons.

**14.** Alcoolates de métaux de terre rare suivant la revendication 3 ou 7, caractérisés en ce que pour augmenter l'indice de coordination, au moins l'un des radicaux $R_c$ organiques est constitué en tant que "bras donneur" en ayant la structure suivante :

$CR_aR_b$-Do-$R_{Do}$, en utilisant la nomenclature de la revendication 1, $R_a$ et $R_b$ étant des radicaux organiques exempts de donneurs conformément à la revendication 1.

**15.** Alcoolates de métaux de terre rare suivant la revendication 14, caractérisés en ce que, pour augmenter encore l'indice de coordination, il est prévu un autre radical $R_c$ organique servant de "bras donneur" ayant la structure suivante :

- $(CH_2)_n$-Do-$R_{Do}$, dans laquelle $n \geq 2$.

**16.** Alcools destinés à la synthèse de composés volatils, notamment d'alcoolates, correspondant à l'une des formules structurales suivantes :

$HOC^tBU_2CH_2OEt$
$HOC^iPr_2CH_2OEt$

**17.** Procédé de préparation de couches minces en oxyde de métaux de terre rare, caractérisé par un procédé MOCVD sans réaction en utilisant des alcoolates de métaux de terre rare volatils suivant les revendications 1 à 15.

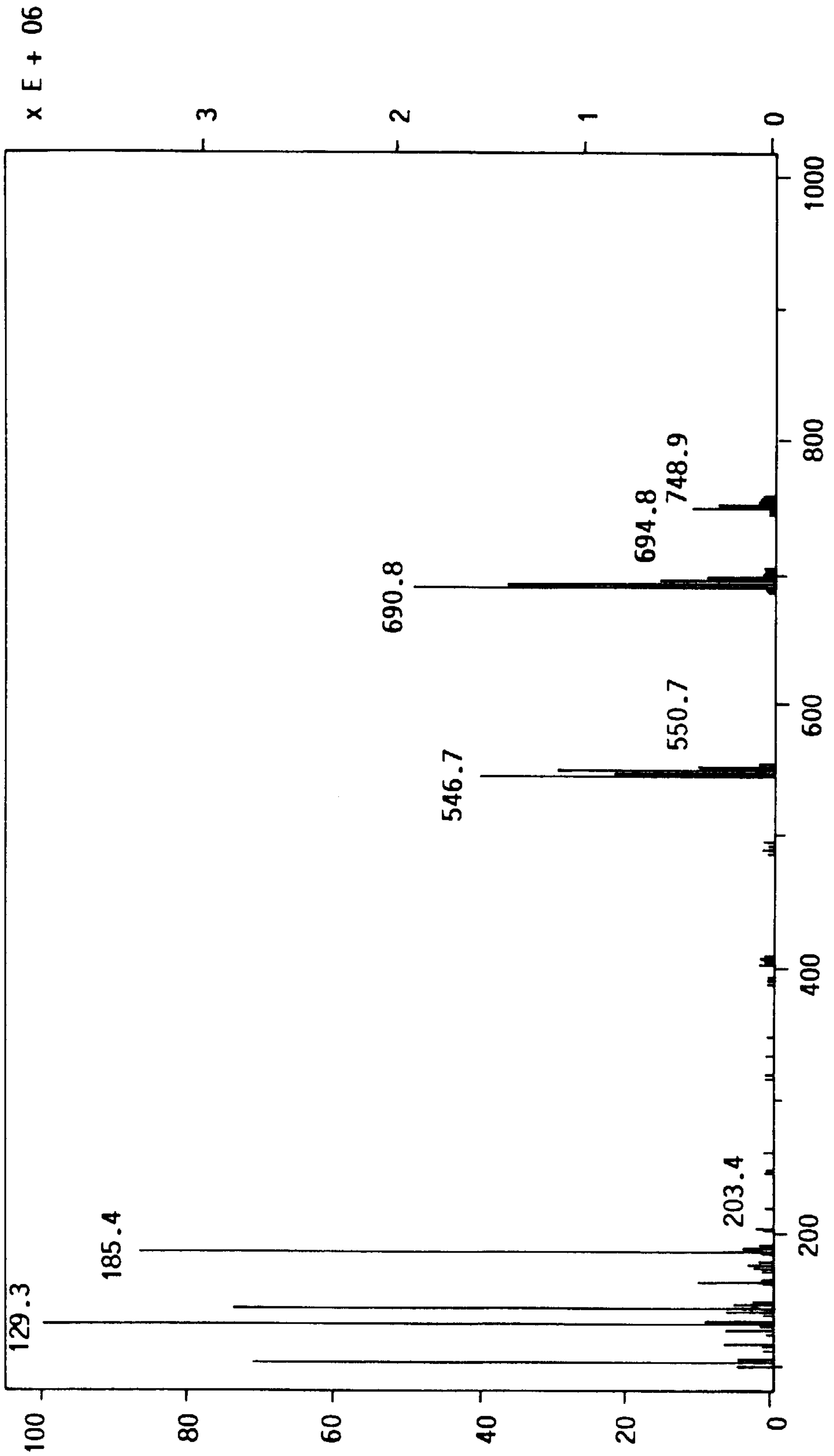
x E + 06
3
2
1
0
129.3
185.4
203.4
546.7
550.7
690.8
694.8
748.9
100
80
60
40
20
0
200
400
600
800
1000

FIG. 1

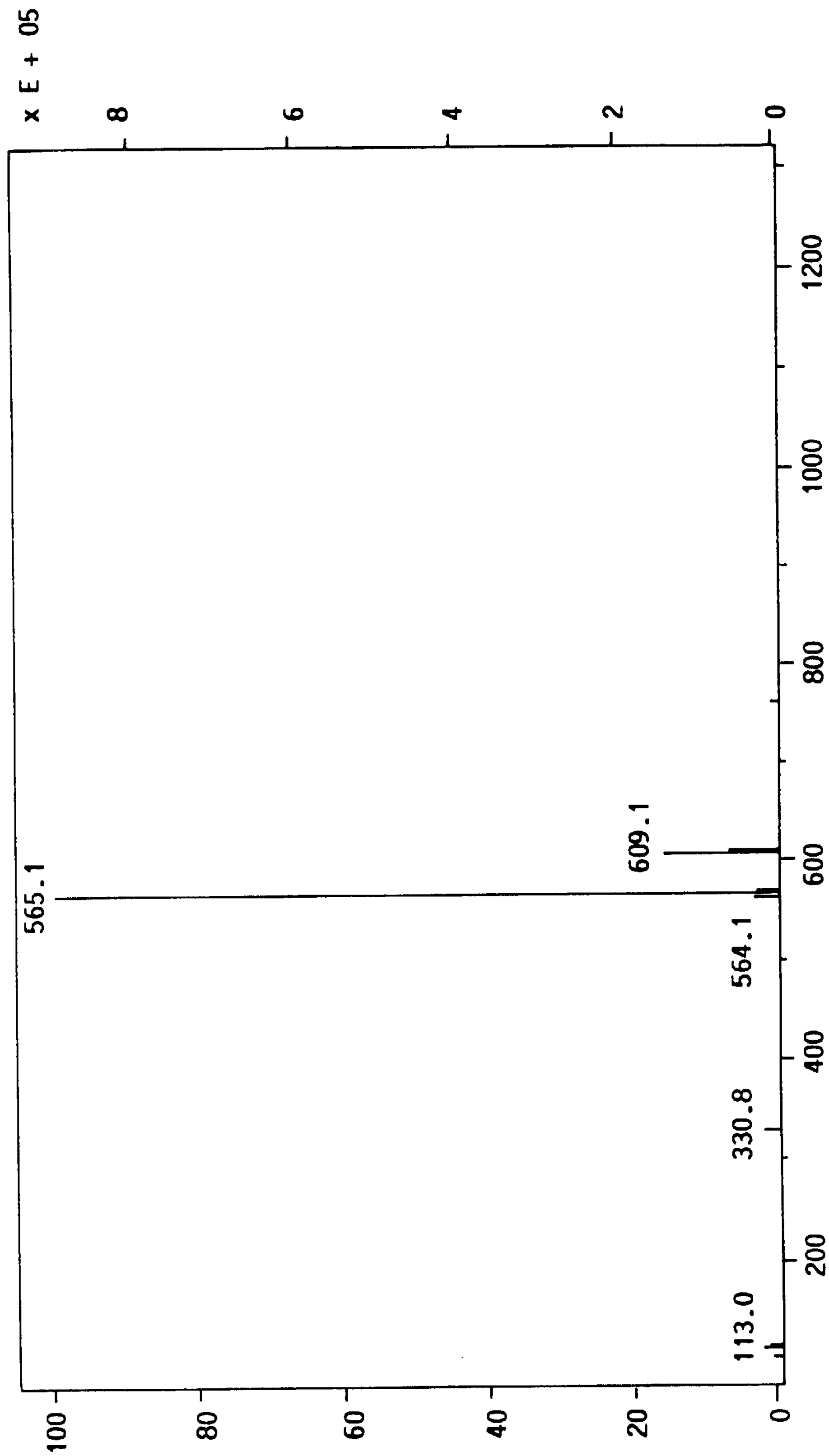
x E + 05
8
6
4
2
0
100
80
60
40
20
0
200
400
600
800
1000
1200
565.1
609.1
564.1
330.8
113.0

FIG. 2